Europäisches Patentamt

European Patent Office

Office européen des brevets

(11) Publication number: **0 024 941**
**B1**

# EUROPEAN PATENT SPECIFICATION

(45) Date of publication of patent specification: **19.03.86**

(21) Application number: **80303042.8**

(22) Date of filing: **01.09.80**

(51) Int. Cl.⁴: **C 12 P 1/04,** A 61 K 35/74 //
C12R1/15

(54) **Water-soluble extract from nonpathogenic aerobic corynebacteria, production thereof and compositions containing the extract.**

(30) Priority: **30.08.79 JP 110724/79**

(43) Date of publication of application:
**11.03.81 Bulletin 81/10**

(45) Publication of the grant of the patent:
**19.03.86 Bulletin 86/12**

(84) Designated Contracting States:
**AT BE CH DE FR GB IT LI NL SE**

(56) References cited:
FR-A-2 218 888
FR-A-2 410 476
GB-A-1 502 320

CHEMICAL ABSTRACTS, vol. 87, no. 7, 15th
August 1977, page 339, abstract 51627p,
COLUMBUS OHIO (US)

CHEMICAL ABSTRACTS, vol. 49, no. 1, 10th
January 1955, page 421, column 421b,
COLUMBUS OHIO (US)

The file contains technical information
submitted after the application was filed and
not included in this specification

(73) Proprietor: **YAMANOUCHI PHARMACEUTICAL
CO. LTD.**
**No. 5-1 Nihonbashi-Honcho, 2-chome Chuo-ku
Tokyo (JP)**

(72) Inventor: **Sindo, Tiuzi**
**57-13, Denenchofu-Honmachi Ota-ku
Tokyo (JP)**
Inventor: **Tadashi, Obara**
**No.1007, Ochiaipakufamiria No.1-1-15,
Kamiochiai
Shinjuku-ku Tokyo (JP)**
Inventor: **Adachi, Hidenari**
**No. 16-6, Shinmori 5-chome Asahi-ku
Osaka-shi Osaka (JP)**

(74) Representative: **Geering, Keith Edwin et al
REDDIE & GROSE 16 Theobalds Road
London WC1X 8PL (GB)**

(56) References cited:
**CHEMICAL ABSTRACTS, vol. 89, no. 5, 31st
July 1978, page 418, abstract 40771w,
COLUMBUS OHIO (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to a water-soluble extract from nonpathogenic aerobic corynebacteria.

It has been reported (GB 1502320A) that the cultured broth (bacterial cells) of anaerobic corynebacteria such as Corynebacterium parvum, and extracts from the cutured cells, have an anti-cancer effect and a protective effect against infection.

The composition of organic solvent-soluble extracts from the ectoplasm of whole aerobic corynebacteria cells has been investigated (Chem. Abs., Vol. 49, No. 1, column 421 b) but no pharmacological activity noted therefor.

It has been found that whole bacterial cells obtained by cultivating bacterial strains of nonpathogenic aerobic corynebacteria had not only an anti-cancer effect and an immunopotentiating effect for spleen cells and lymphocytes but also an anti-allergic effect which had not been known for cultured anaerobic corynebacteria (JP—A—77 47909; Chem. Abs., Vol. 89, No. 5, p. 40768, abstract 40771 w). However, the cultured broth is in practice unsuitable for medicinal use. Thus the cultured bacterial cells are insoluble in water, and so must be administered in the form of a suspension; when this suspension is administered subcutaneously, it causes at the injection site induration like that caused by subcutaneous injection of BCG, making multiple administrations intolerable. Furthermore, the cultured broth (bacterial cells) shows strong toxicity.

We have now found that there can be obtained from nonpathogenic areobic corynebacteria a water soluble extract which has useful pharmacological activity and can be used satisfactorily as a medicament.

The detailed composition of such active water soluble extracts according to this invention is not clear, but appear to be glycoprotein materials containing protein, sugar, and nucleic acid-like material.

Preferred water soluble extract according to the invention, has a protein content (calculated as Bovine Serum Albumin) of 3.9 to 5.5 mg/ml, a sugar content (calculated as glucose) of 0.41 to 0.52 mg/ml, and an isoelectric point of 3.75 to 3.80, the ratio of the optical density of the extract at 260 nm to that at 280 nm being from 1.56 to 1.62.

Water soluble extracts according to this invention have shown anti-cancer, immunopotentiative and anti-allergic effects, with weak toxicity as compared with that of the primarily cultured broth. Furthermore, since the active materials provided by this invention are soluble in water, multiple doses can be administered by subcutaneous or intradermal injection without causing induration of the skin; hence they can be used for desensitization therapy which is an important therapy for allergic diseases.

Strains of nonpathogenic aerobic corynebacteria suitable for use in the invention include the Corynebacterium equi Ko-85, Corynebacterium pyogenes Tojo Hai, Corynebacterium xerosis 53-K-1, and Corynebacterium renale Utsunomiya-Ushi strains. These strains are readily available to persons skilled in the art and are also preserved in the Medical Research Insitute of Tokyo University as standard strains. The particularly preferred bacterial strain for use in this invention is the Corynebacterium equi Ko-85 strain. The cultured bacterial cells can be obtained by aerobic culture of the corynebacteria in conventional manner, e.g. as described in the Reference Example below.

A water soluble extract can be obtained from the primary broth by subjecting the primarily cultured broth to low temperature, grinding and homogenization, subjecting the homogenate to ultrasonic treatment to separate effective components which are precipitated by adjusting the pH of an aqueous solution thereof (usually to 3.7—3.75), forming an almost neutral aqueous solution of the precipitate, and dialyzing this aqueous solution; the extract obtained can be converted into a powdery solid product by lyophilization. The individual steps can be practised conventionally. The resulting water soluble extract can have anti-allergic, anti-cancer, and immunopotentiative effects, and may be particularly useful as an anti-allergic agent.

Pharmaceutical compositions can contain water soluble extract according to the invention in combination with at least one compatible and pharmaceutically acceptable diluent or excipient. The pharmaceutical compositions of this invention may be administered by oral, rectal or parenteral routes or by aerosol. In human therapy, the dosage is a function of the desired effect. The dosages may for example be 10 ng to 500 µg per day for an adult patient.

The invention is illustrated by the following non-limiting Examples and accompanying drawings.

The reference Example describes a production of primarily cultured broth (bacterial cells), and Production Examples 1 and 2 the formation of extracts according to the invention from this cultured broth. In the drawings:

Figure 1a is a graph showing the elution pattern of a Production Example 1 extract using Sephadex G-200 Gel,

Figure 1b is a graph showing the elution pattern of a Production Example 1 extract using Ultro Gel ACA 34,

Figure 2 is a graph showing the suppressive effects on reaginic antibody production of an extract according to the invention,

Figure 3 illustrates cross reactive anti-genicities by gel precipitation (Ouchterloney method), and

Figure 4 is a graph showing the anti-complement effect of an extract according to this invention.

Reference Example

Corynebacterium equi Ko-85 strain was cultivated at 37°C for 5 days using a culture medium prepared by adjusting a mixture of 10 g. of a meat extract, 10 g. of peptone, 10 g. of glucose, 1 g. of sodium chloride, and 1000 ml of redistilled water to pH 7.4 with an aqueous sodium hydroxide solution. Then, after continuous centrifugation at a throughput speed of 50 ml/min. and at 10,000 r.p.m., the precipitated bacterial cells formed were washed centrifugally three times with an isotonic sodium chloride solution to provide a primarily cultured broth (bacterial cells).

The yield of cultured broth (bacterial cells) obtained was 17.0 g (wet weight) per 10 liters of the culture medium used.

The relation between the yield of cultured broth (by turbidimetric determination) and the culture period was as shown below, the yield being at a maximum after about 120 hours cultivation.

| Culture time (hours) | Optical density of corynebacterium equi Ko-85 |
|---|---|
| 0 | 0.175 |
| 4 | 0.165 |
| 20 | 0.320 |
| 30 | 0.400 |
| 50 | 0.720 |
| 70 | 0.770 |
| 120 | 1.075 |
| 1 week | 1.070 |

Production Example 1

50 g of the primarily cultured broth from the Reference Example were washed with an isotonic sodium chloride solution, and then subjected to a low-temperature treatment for longer than 48 hours at −20°C and the bacterial cells were ground and homogenized by Vibrogen cell mill (VCH-1). The homogenate formed was suspended in water or a dilute aqueous sodium hydroxide solution and after six ultrasonic treatments, each at 20 kHz for 10 minutes in ice water, the suspension was extracted with shaking. Thereafter, 150 g of the extract obtained was centrifuged for 60 minutes at 105,000 g to remove bacterial cell residues and insoluble matter.

The supernatant formed was adjusted to a concentration corresponding to 25% wet bacterial cells and after adjusting the pH thereof to 3.7—3.75 using 1N hydrochloric acid at 8—10°C, the precipitates were incubated over 48 hours. The precipitates were collected by centrifuging, dissolved in water at pH 7.2, the solution was dialyzed overnight, and after adjusting the pH of the system to 7.0, the system was subjected to centrifugal separation for 60 minutes at 12,000 g to form a supernatant (extract), which was lyophilized. The yield of the product was 500 mg (dry weight).

The physicochemical characteristics of the product were as follows:

A. Properties

The extract was a light yellow-brown transparent liquid.

When the pH of the extract was adjusted to 3.75—3.80, precipitates formed.

B. Composition

Three test samples (extracts) were separately prepared in the manner described above and designated as No. 1, No. 2, and No. 3 respectively. The compositions of these test samples, and methods of determining composition, were as follows:

# 0 024 941

|  | No. 1 | No. 2 | No. 3 |
|---|---|---|---|
| Protein content[*1] (mg/ml) | 4.3 | 3.9 | 5.5 |
| Sugar content[*2] (mg/ml) | 0.46 | 0.41 | 0.52 |
| Nucleic acid-like substance[*3] (OD 280 nm) | 1.13 | 0.90 | 1.10 |
| Nucleic acid-like substance[*3] (OD 260 nm) | 1.83 | 1.42 | 1.72 |
| Nucleic acid-like substance[*3] (260 nm/280 nm) | 1.62 | 1.58 | 1.56 |
| Isoelectric point (pH) | 3.75 | 3.75 | 3.80 |

[*1]:Protein content (copper-folin method)

The extract was diluted with water to 10 times the volume of the extract and after adding 3 ml of solution C shown below to 0.6 ml of the dilution, they were reacted for 10 minutes. After the reaction was over, 0.3 ml of a phenol solution (prepared by diluting a commercially available product with water) was added to the reaction mixture and after allowing the mixture to stand for 30 minutes at room temperature, the optical density (O.D.) at 500 nm was measured. A standard curve for Bovine Serum Albumin (BSA) was made over a concentration range of 0.2—1.0 mg/ml, and from the standard curve the content in the extract was determined. Solution C was obtained from A and B as follows:

Solution A: To a 0.1 N sodium hydroxide solution was added sodium carbonate at a concentration of 2%.

Solution B: To a 10% sodium citrate solution was added copper sulfate at a concentration of 0.5%.

Solution C: A mixture of 50 ml of solution A and 1 ml of solution B (50:1).

[*2]:Sugar content (phenol sulfuric acid method)

The extract was diluted with water to 10 times the volume of the extract and then 1 ml of a 5% phenol solution and 5 ml of sulfuric acid were immediately added to 1 ml of the dilution. After allowing the mixture to stand for 20 minutes, the optical density (O.D.) at 490 nm was measured. A standard curve for an aqueous glucose solution was made over a concentration range of 0.02—0.2 mg/ml, and from the standard curve the content in the extract was determined.

[*3]:Nucleic acid-like substance content

The extract was diluted with water to 20 times the volume of the extract, the optical densities (O.D.) at 260 nm and 280 nm were measured, and the ratio of them was determined.

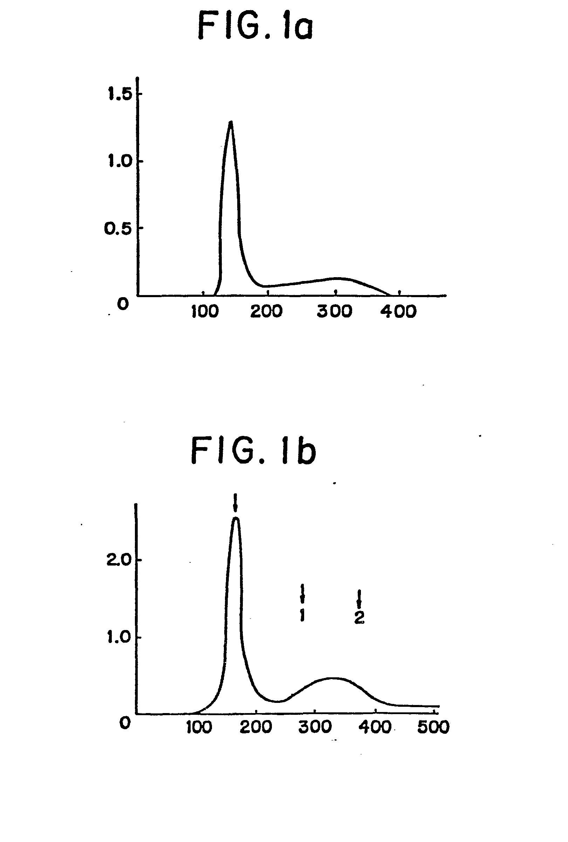

The elution pattern of an extract of this Example with Sephadex G-200 gel shows a single peak as shown in Figure 1a.

The bed volume was 3.0×60 cm², the elution buffer was a 0.01 M phosphate buffer, and the flow rate was 20.8 ml/hour.

The ordinate shows optical density at 280 nm and the abscissa shows elution volume (ml).

The elution pattern of an extract of this Example with Ultrogel ACA 34 is shown in Figure 1b.

The column size was 2.8×84 cm², the elution buffer was a 0.01 M phosphate buffer (containing 0.85% sodium chloride), and the flow rate was 14.0 ml/hour. The ordinate shows optical density at 280 nm, the abscissa shows elution volume (ml), the arrow 1 shows the position of bovine α- globulin (molecular weight: 160,000) and the arrow 2 shows the position of soybean trypsin inhibitor (molecular weight: 21,700).

Production Example 2

After subjecting 152 g of the primarily cultured broth (bacterial cells) to a low-temperature treatment for 48 hours at −20°C, the cultured broth was suspended in distilled water together with glass beads of 0.1 mm diameter and the suspension was treated 10 times in a Vibrogen cell mill (VCH-1) at 4500 r.p.m. for 30 minutes at 0°C. to grind and homogenize the bacterial cells. Then, after centrifuging the supernatant formed for 10 minutes at 1,000 r.p.m., the supernatant was subjected to an ultrasonic treatment at 20 kHz for 15 minutes in ice water and then centrifuged for 20 minutes at 3000 r.p.m. and then for 40 minutes at 16,000 r.p.m. (20,000 g) to remove bacterial cell residues and insoluble materials. The supernatant was adjusted to a concentration corresponding to 25% wet bacterial cells, the pH thereof was adjusted to 3.70 using a 1N hydrochloric acid at 4°C, and it was stood overnight to complete the precipitation. The precipitates were collected by centrifugation for 30 minutes at 5,000 r.p.m. and dissolved in distilled water after adjusting the pH thereof to 7.0 with 1N sodium hydroxide, the solution was dialyzed overnight with distilled water at 4°C and then for 2 days with an isotonic sodium chloride solution at the same

4

**0 024 941**

temperature; after adjusting the pH to 7.0 and centrifugation for 40 minutes at 16,000 r.p.m. (20,000 g), the supernatant was filtered by means of a membrane filter (Millipore HA) to provide about 500 ml of an extract, which was then lyophilized.

The results of pharmacological and toxicity tests on the extracts of this invention are shown below:

### A. Suppressive effect on immunoglobulin E (IgE) antibody

In an isotonic sodium chloride solution were suspended 2 µg of DNP-OA together with an aluminum hydroxide gel and the suspension was administered to BALB/c mice by intraperitoneal injection; 600 µg of an extract of this invention was administered 2 and 7 days after immunization. Sera were collected from animals on days 5, 10, 15, 20, 25 and 30 after immunization and were combined to provide respective pools, and the pooled sera were used for sensitization of the skins of the hind back portions of SD rats. 4 hours after sensitization, a solution of antigen DNP-BSA containing 0.5% Evans blue was injected into the tail veins, and the IgE titre was measured by the amount of dye extracted from the reaction site.

In two comparison runs, the above procedure was repeated but with the DNP-OA sensitized BALB/c mice being inoculated (in each case 2 and 7 days after sensitization) respectively with (a) killed bacterial cells of aerobic Corynebacterium parvum and (b) a physiological sodium chloride solution instead of with the extract of this invention.

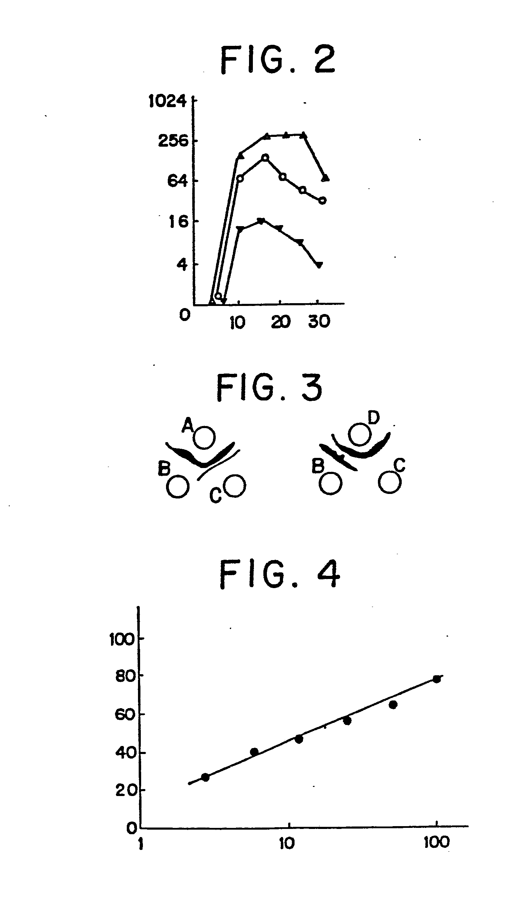

Figure 2 shows the results obtained with the extract of the invention (▼——▼), with said killed bacterial cells (Δ——Δ), and with said physiological solution (○——○).

The Figure 2 ordinate show IgE antibody titre and the abscissa shows days after the DNP-OA sensitization.

From the results shown in Figure 2, it is clear that the extract of this invention suppresses the IgE antibody production which triggers an immediate type of allergy.

### B. Suppressive effect on passive cutaneous anaphylaxes (PCA) by local treatment

The skins of SD rats were previously treated with extract of this invention; 2 hours later the rats were inoculated with anti-DNP (hapten) IgE antibody at the same area of the skins, and passive cutaneous anaphylaxes (PCA) were done to detect the suppressive effect.

The results are shown in Table IA.

### TABLE IA

| Extract of the invention (mg) | Amount of dye extracted (µg) dilution of antibody solution | | | | | |
|---|---|---|---|---|---|---|
| | 1/8 | 1/16 | 1/32 | 1/64 | 1/128 | 1/256 |
| 5.0 | 11.2 (29) | 12.0 (19) | 11.8 (19) | 9.4 (17) | 7.5 (10) | — |
| | 11.3 (28) | 10.7 (28) | 12.7 (13) | — (100) | — | — |
| 2.5 | 13.1 (17) | 12.6 (15) | 11.5 (21) | 7.7 (32) | — | — |
| | 12.1 (23) | 12.8 (14) | 11.6 (21) | 7.2 (36) | — | — |
| 1.0 | 16.8 (0) | 14.6 (2) | 15.0 (0) | 10.0 (12) | 6.5 (22) | trace |
| | 15.2 (3) | 15.4 (0) | 14.8 (0) | 10.1 (11) | — | — |
| physiological sodium chloride soln. | 15.7 | 14.9 | 14.6 | 11.3 | 8.3 | trace |

(NOTE): In the above Table, the numerals in parentheses show the suppressive rates on PCA reaction.

As shown in the Table, the extract of this invention blocks binding of IgE antibody on target cells (mast cells, basophils, etc.,) and suppresses the liberation of histamine on antigen invasion.

# 0 024 941

C. Suppressive effect on passive cutaneous anaphylaxes (PCA) by systemic treatment.

The PCA suppressive effect was more remarkable when treating with extract of this invention before the IgE antibody sensitization than when so treating after the sensitization. The results are shown in the following Table, in which the amount of extract of this invention used for the treatment was 1.0 mg and the PCA value was shown by the amount of dye extracted.

TABLE I—B

| Treatment time of extract of the invention (time before or after antibody sensitization) | Amount of dye extracted (μg) dilution of antibody solution | | |
|---|---|---|---|
| | 1/20 | 1/40 | 1/80 |
| −24 | 27.3±8.1 (60) | 16.0±6.5 (54) | 6.5±4.3 (47) |
| −8 | 14.3±4.8 (32) | 8.3±3.8 (28) | 2.4±1.1 (17) |
| −2 | 20.7±8.8 (46) | 12.3±6.9 (42) | 5.0±3.1 (36) |
| 0 | 32.0±11.3 (71) | 21.5±11.5 (73) | 12.0±8.5 (87) |
| +2 | 43.1±8.1 (95) | 29.4±13.1 (100) | 11.9±7.9 (86) |
| +4 | 40.9±10.0 (90) | 28.6±11.9 (97) | 10.0±6.4 (72) |
| Control | 45.3±12.9 | 29.4±10.8 | 13.8±8.3 |

(NOTE): The numerals in parentheses show the ratios compared with the control.

The above described experimental results show that the extract of this invention inhibits passive cutaneous anaphylaxes elicited by mast cells or basophils by binding of IgE antibody, which was sensitized with antigen, regardless of the kind of antigen, and also suppresses allergic reaction in a wide range thereafter.

D. Increase in production of antihapten IgE, (IgM) antibody

Extract of this invention was intravenously injected into BALB/c mice 7 weeks old in an amount of 0.1 mg, and the mice were intravenously sensitized with 100 μg of DNP-OA 7 and 14 days after immunization. 3 days later, the number of antibody forming cells in the spleen cells was measured by Cunningham's indirect method using DNP-BSA sensitized sheep erythrocytes. The results are shown in Table II.

6

0 024 941

## TABLE II
### Increase of number of antihapten IgG, (IgM) antibody forming cell

| No. of tested mouse | | 1 | 2 | 3 | 4 | 5 | Mean value (± standard deviation) |
|---|---|---|---|---|---|---|---|
| The water soluble extract of this invention | Number of haemolytic plaques in an observation field | 240.3 | 229.5 | 230.5 | 117.0 | 119.0 | 187.8±63.9 |
| | Number of haemolytic plaques / $10^6$ nucleated spleen cells | 147.8 | 141.2 | 141.9 | 72.0 | 73.0 | 115.5±39.4 |
| | Total number of haemolytic plaques per mouse | 6847 | 7738 | 7180 | 3240 | 3862 | 5773±2065.4 |
| Primarily cultured broth (bacterial cells) | Number of haemolytic plaques in an observation field | 212.5 | 192.5 | 208.0 | 209.0 | 202.0 | 204.8±7.9 |
| | Number of haemolytic plaques / $10^6$ nucleated spleen cells | 130.8 | 118.5 | 134.8 | 130.5 | 120.0 | 126.9±7.2 |
| | Total number of haemolytic plaques per mouse | 6344 | 7110 | 7144 | 7021 | 6318 | 6818±329.3 |
| Control (normal saline solution) | Number of haemolytic plaques in an observation field | 17.0 | 45.5 | 16.5 | 18.0 | 46.5 | 28.7±15.8 |
| | Number of haemolytic plaques / $10^6$ nucleated spleen cells | 10.5 | 28.0 | 10.2 | 11.1 | 28.6 | 17.7±9.7 |
| | Total number of haemolytic plaques per mouse | 430 | 1344 | 498 | 475 | 1224 | 794±449.9 |

As shown in Table II, it is clear that the extract of this invention increases the production of antihapten IgG (and IgM) antibody. Therefore, it is also clear that the extract of this invention possesses an immuno-activation effect and the production of anti-bodies other than allergic antibody is not reduced although it may be accelerated.

E. Antigenic cross reactivity

The antigenic cross reactivity was determined by a gel precipitation reaction (Ouchterloney method).

Using rabbit anti-Corynebacterium equi anti serum and rabbit anti-tuberculin anti serum, a gel precipitation reaction was performed according to the Ouchterloney method with extract of this invention and PPD solution (TA₂), each adjusted to 10 mg/ml, as antigens. The results are shown in Figure 3.

Figure 3 shows the fused precipitation lines of PPD solution (B) and the extract of this invention (C) against anti-Corynebacterium equi anti serum (A) in the left section and also the cross reactive anti-genicity to the PPD solution (B) and to the extract of this invention (C) by the rabbit anti-tuberculin anti serum (D) in the right section.

From the results shown in Figure 3, it is clear that the extract of this invention possesses antigenic cross reactivity with PPD. Thus, the extract of this invention can be applied to tuberculin therapy in more stable form.

F. Anticomplement effect

After reacting the sera of SD rats with extract of this invention at various concentrations for 1 hour at 37°C, the residual haemolytic activity of complement was measured using sheep erythrocytes sensitized by anti-SRBC antibody. The results are shown in Figure 4, in which the abscissa shows the added amount (μg) of the extract of this invention and the ordinate shows the percentage inhibition of the haemolytic reaction.

As is clear from Figure 4, it was confirmed by the inhibition of the haemolytic reaction with sheep erythrocytes that the extract of this invention had remarkable anticomplement effect. It thus appears that the extract of this invention can suppress non-specific allergic reaction.

G. Toxicity

The acute toxicity (in the case of intrapertoneal administration) of extract of this invention and of primarily cultured broth (bacterial cells) therefor is as follows. Because the primarily cultured broth is insoluble in water, it was administered as an aqueous suspension.

| | | |
|---|---|---|
| Extract of the invention | $LD_{50}$ | >20 mg/mouse |
| Primarily cultured broth (bacterial cells) | $LD_{50}$ | 5 mg/mouse |

The extract of this invention thus shows very weak toxicity as compared with the primarily cultured broth (bacterial cells) for the extract.

In tests on the effect on peripheral leukocyte counts and the effect on body weight gain in mice by the general test methods of "Minimum Requirement of Biological Products" (1973); Minist. of Health and Welfare, Japan. "-3 General Testing Method-", extract of this invention showed no abnormality.

Prescription Example

An aqueous solution (5 mg/ml) of extract (lyophilized product) of this invention was prepared, diluted to 100 times the volume of the aqueous solution with isotonic sodium chloride solution, and 1 ml lots of the diluted solution were filled into ampoules.

For desensitizing therapy the prescription may be injected subcutaneously in an amount of 0.1—0.8 ml per injection once or twice per week.

**Claims**

1. A water soluble extract from nonpathogenic aerobic corynebacteria, the extract having a protein content (calculated as Bovine Serum Albumin) of 3.9 to 5.5 mg/ml, a sugar content (calculated as glucose) of 0.41 to 0.52 mg/ml, and an isoelectric point of 3.75 to 3.80, the ratio of the optical density of the extract at 260 nm to that at 280 nm being from 1.56 to 1.62.

2. An extract according to claim 1 wherein the corynebacteria comprise Corynebacterium equi Ko-85 strain.

3. An extract according to claim 1 wherein the corynebacteria comprise at least one of the Corynebacterium pyogenes Tojo Hai, Corynebacterium xerosis 53-K-1 and Corynebacterium renale Utsunomiya-Ushi strains.

4. A method of producing a water soluble extract according to claim 1 which comprises subjecting a cultured broth obtained by aerobically cultivating nonpathogenic aerobic corynebacteria to low-temperature treatment, grinding and homogenization, removing insoluble matter from the homogenate, adjusting the pH of an aqueous solution of the remaining active component thus obtained to cause precipitation, forming a neutral or almost neutral aqueous solution of the precipitate obtained, and dialyzing this solution.

5. A method according to claim 4 wherein the nonpathogenic aeerobic corynebacteria comprise at least one of Corynebacterium equi Ko-85, Corynebacterium pyogenes Tojo Hai, Corynebacterium xerosis 53-K-1 and Corynebacterium renale Utsunomiya-Ushi strains.

6. A method according to claim 4 or 5 including lyophilizing the dialyzed solution to give a powder product.

7. A method according to any of claims 4 to 6 wherein the ground and homogenized cultured broth is subjected to an ultrasonic treatment, and the pH of the said aqueous solution of the active component is adjusted to 3.7—3.75 to cause the precipitation.

8. A method according to any of claims 4 to 7 including adding water to the product.

9. A lyophilizate of an extract according to any of claims 1 to 3.

10. A dry powder obtainable by lyophilisation of an extract according to any of claims 1 to 3.

11. An aqueous solution obtainable by adding water to an extract according to any of claims 1 to 3 or to a product according to claim 9 or 10.

12. A pharmaceutical composition containing as an active ingredient a product according to any one of claims 1 to 3 and 9 to 11.

**Patentansprüche**

1. Ein wasserlöslicher Extrakt aus nichtpathogenen, aeroben Corynebakterien, gekennzeichnet durch einen Proteingehalt (berechnet als Rinderserum-Albumin) von 3,9 bis 5,5 mg/ml, einem Zuckergehalt (berechnet als Glucose) von 0,41 bis 0,52 mg/ml, und einem isoelektrischen Punkt von 3,75 bis 3,80 und durch das Verhältnis der optischen Dichte des Extraktes bei 260 nm zu den bei 280 nm zwischen 1,56 und 1,62.

2. Ein Extrakt nach Anspruch 1, dadurch gekennzeichnet, daß die Corynebakterien den Stamm Corynebakterium equi Ko-85 enthalten.

3. Ein Extrakt nach Anspruch 1, dadurch gekennzeichnet, daß die Corynebakterien wenigstens einen der Stämme Corynebakterium pyogenes Tojo Hai, Corynebakterium xerosis 53-K-1 und Corynebakterium renale Utsunomiya-Ushi umfaßt.

4. Ein Verfahren zum Herstellen eines wasserlöslichen Extraktes nach Anspruch 1, das die Behandlung einer Kulturbrühe, die durch aerobes Kultivieren nichtpathogener, aerober Corynebakterien erhalten ist, bei einer niederen Temperatur, das Zerreiben und Homogenisieren, das Entfernen unlöslicher Anteile aus dem Homogenisat, das Einstellen des pH-Wertes einer wässerigen Lösung der so erhaltenen, zurückbleibenden Wirkkomponente, um eine Ausfällung herbeizuführen, das Bilden einer neutralen oder nahezu neutralen, wässerigen Lösung des erhaltenen Niederschlages, und das Dialysieren dieser Lösung umfaßt.

5. Ein Verfahren nach Anspruch 4, bei welchem die nicht pathogenen, aeroben Corynebakterien wenigstens einen der Stämme von Corynebakterium equi Ko-85, Corynebakterium pyogenes Tojo Hai, Corynebakterium xerosis 53-K-1 und Corynebakterium renale Utsunomiya-Ushi umfassen.

6. Ein Verfahren nach Anspruch 4 oder 5, welches das Lyophilisieren der dialysierten Lösung zu einem pulverförmigen Produkt einschließt.

7. Ein Verfahren nach einem der Ansprüche 4 bis 6, bei dem die zerriebene und homogenisierte Kulturbrühe einer Ultraschallbehandlung unterzogen wird und daß der pH-Wert der wässerigen Lösung der Aktivkomponente auf 3,7—3,75 eingestellt wird, um das Ausfällen auszulösen.

8. Ein Verfahren nach einem der Ansprüche 4 bis 7, bei dem man dem Produkt Wasser zusetzt.

9. Ein Lyophilisat eines Extraktes nach einem der Ansprüche 1 bis 3.

10. Ein trockenes Pulver, erhältlich durch Lyophilisieren eines Extraktes nach einem der Ansprüche 1 bis 3.

11. Eine wässerige Lösung, erhältlich durch Zugabe von Wasser zu einem Extrakt nach einem der Ansprüche 1 bis 3 oder zu einem Produkt nach Anspruch 9 oder 10.

12. Eine pharmazeutische Zusammensetzung, die als Wirkbestandteil ein Produkt nach einem der Ansprüche 1 bis 3 und 9 bis 11 enthält.

**Revendications**

1. Extrait hydrosoluble de corynébactéries aérobies non pathogènes, l'extrait ayant une teneur en protéines (calculée en albumine sérique bovine) de 3,9 à 5,5 mg/ml, une teneur en sucre (calculée en glucose) de 0,41 à 0,52 mg/ml et un point isoélectrique de 3,75 à 3,80, le rapport de la densité optique de l'extrait à 260 nm à celle à 280 nm étant de 1,56 à 1,62.

2. Extrait selon la revendication 1, dans lequel la corynébactérie est constituée par la souche Corynebacterium equi Ko-85.

3. Extrait selon la revendication 1, dans lequel la corynébactérie est constituée par au moins une des souches Corynebacterium pyogenes Tojo Hai, Corynebacterium xerosis 53-K-1 et Corynebacterium renale Utsunomiya-Ushi.

4. Procédé de préparation d'un extrait hydrosoluble selon la revendication 1, qui consiste à soumettre

9

un bouillon de culture, obtenu par culture aérobie de corynébactéries aérobies non pathogènes, à un traitement à basse température, à un broyage et à une homogénéisation, à éliminer la matière insoluble du produit d'homogénéisation, à ajuster le pH d'une solution aqueuse du constituant actif restant ainsi obtenue pour provoquer la précipitation, à former une solution aqueuse neutre ou presque neutre du précipité obtenu et à dialyser cette solution.

5. Procédé selon la revendication 4, dans lequel la corynébactérie aérobie non pathogène est constituée par au moins une des souches Corynebacterium equi Ko-85, Corynebacterium pyogenes Tojo Hai, Corynebacterium xerosis 53-K-1 et Corynebacterium renale Utsunomiya-Ushi.

6. Procédé selon l'une des revendications 4 et 5, comprenant la lyophilisation de la solution dialysée pour donner un produit en poudre.

7. Procédé selon l'une quelconque des revendications 4 à 6, dans lequel on soumet le bouillon de culture broyé et homogénéïsé à un traitement ultrasonique et on ajuste le pH de la solution aqueuse du constituant actif à 3,7—3,75 pour provoquer la précipitation.

8. Procédé selon l'une quelconque des revendications 4 à 7, comprenant l'addition d'eau au produit.

9. Lyophilisat d'un extrait selon l'une quelconque des revendications 1 à 3.

10. Poudre sèche que l'on peut obtenir par lyophilisation d'un extrait selon l'une quelconque des revendications 1 à 3.

11. Solution aqueuse que l'on peut obtenir en ajoutant de l'eau à un extrait selon l'une quelconque des revendications 1 à 3 ou à un produit selon la revendication 9 ou 10.

12. Composition pharmaceutique contenant comme ingrédient actif un produit selon l'une quelconque des revendications 1 à 3 et 9 à 11.

# FIG. 1a

# FIG. 1b

# FIG. 2

# FIG. 3

# FIG. 4